# EUROPEAN PATENT SPECIFICATION

(11) **EP 3 273 374 B1**
(45) Date of publication and mention of the grant of the patent: **17.05.2023**
(21) Application number: 17172462.8
(22) Date of filing: 23.05.2017
(51) Int. Cl.: G16H 30/40, G06T 7/00, G06F 16/583, G16H 50/20, G06F 9/455, G16H 10/60, G16H 40/40, G06F 8/76

(54) **METHOD FOR ANALYSING IMAGE DATA OF A PATIENT, SYSTEM FOR PERFORMING A METHOD FOR ANALYSING IMAGE DATA OF A PATIENT AND COMPUTER READABLE MEDIUM**
VERFAHREN ZUR ANALYSE VON BILDDATEN EINES PATIENTEN, SYSTEM ZUR AUSFÜHRUNG EINES VERFAHRENS ZUR ANALYSE VON BILDDATEN EINES PATIENTEN UND COMPUTERLESBARES MEDIUM
PROCÉDÉ D'ANALYSE DE DONNÉES D'IMAGE D'UN PATIENT, SYSTÈME DESTINÉ À EFFECTUER UN PROCÉDÉ D'ANALYSE DE DONNÉES D'IMAGE D'UN PATIENT ET SUPPORT LISIBLE PAR ORDINATEUR

(43) Date of publication of application: 24.01.2018
(73) Proprietor: Siemens Healthcare GmbH, 91052 Erlangen (DE)
(72) Inventor: Schmidt, Sebastian, 91085 Weisendorf (DE)

(56) References cited:
- US-A1- 2006 098 855
- US-A1- 2016 093 045
- Anonymous: "Virtual machine - Wikipedia", , 21 May 2017 (2017-05-21), XP055423050, Retrieved from the Internet: URL:https://web.archive.org/web/2017052108 0317/https://en.wikipedia.org/wiki/Virtual _machine [retrieved on 2017-11-08]
- Anonymous: "Laufzeitumgebung - Wikipedia", , 20 November 2016 (2016-11-20), XP055423051, Retrieved from the Internet: URL:https://web.archive.org/web/2016112016 1026/https://de.wikipedia.org/wiki/Laufzei tumgebung [retrieved on 2017-11-08]

## Description

The present invention deals with a method for analysing image data of a patient, a system for performing a method for analysing image data of a patient and a computer readable medium.

Analysing image data of a patient, in particular analysing image data automatically, is well known from the state of the art. Typically, an analysis algorithm recognises and/or measures a malformation being visible in the image data of the patient. For example, a CT-image is recorded by a corresponding imaging instrument and the analysis of the recorded CT-image is performed by the analysis algorithm, wherein the analysis algorithm outputs a numerical value for the size of the malformation, such as a pulmonary node. For observing a potential growth of the malformation the patient might revisit the hospital several times over a wide time span, wherein each time a new image data set is recorded and analysed.

However, the analysis algorithm and/or operation system of workstations being used for running the analysis algorithm changes over time. Therefore, the same analysis algorithm is not used for several different image data sets of the same patient. Thus, the outputs of the analysis algorithms might not be comparable for different image data sets.

Methods for the analysis of image data are known for example from US 2016/0180052 A1 or US 9,384,413 B2.

US 2016 / 0 093 045 A1 deals with a medical image storage unit that stores a medical image and an image processing unit that performs image processing on a medical image to be stored in the medical image storage unit. Further, the unit stores the result of the image processing in association with the image processing target medical image, and a past medical image identification unit that identifies, at a storage time point of a new storage target medical image in the medical image storage unit or at a time point before the storage time point, a past medical image related to the new storage target medical image from the medical images stored in the medical image storage unit. Thereby, the image processing unit performs the same image processing as that for the new storage target medical image on the identified past medical image and stores the result of the image processing in association with the past medical image.

US 2006 0098855 A1 discloses a method and a system for acquiring, processing, storing, and displaying x-ray mammograms Mp, tomosynthesis images Tr representative of breast slices, and x-ray tomosynthesis projection images Tp taken at different angles to a breast, where the Tr images are reconstructed from Tp images.

It is an object of the present invention to provide a method for analysing several image data sets of a patient, wherein the method yields outputs being comparable.

This object is achieved by a method according to claim 1, by a system according to claim 5 as well as by a computer readable medium according to claim 6. Further advantages and features of the invention result from the dependent claims and the description and the accompanying figures.

In accordance with the invention a method for analysing image data of a patient intended for automatically interpreting and/or categorizing the content of an image data set, is provided:
- in a first step a first image data set of the patient is provided by an imaging instrument being a CT-, NMR-, PET- or X-ray-instrument;
- subsequently, the first image data set is analysed by using a first analysis algorithm at a workstation having a processor for running the first analysis algorithm in a second step;
- afterwards, in a third step, the first image data set is saved in a memory device and a piece of information about the first analysis algorithm is saved along with the first image data set;
- a server having a database for analysis algorithms is provided,
   - wherein the first analysis algorithm and a second analysis algorithm are stored on the database and the first analysis algorithm is available on the server during the first and the second step, while the second analysis algorithm is not available during the first and the second step,
   - and wherein the first analysis algorithm is stored in a closed first container comprising a first run time environment and the second analysis algorithm is stored in a second container comprising a second run time environment,
- the second analysis algorithm is added in a fourth step to the database after the second step and/or the third step,
- for performing the analysis of a second image data set, the second image date set is assigned to the patient in a fifth step;
- based on the piece of information saved along with the first image data set, selecting the first container from the database for analysing the second image data set, wherein the first container is transferred from the server to the workstation in a sixth step, thereby analysing the second image data set with the first analysis algorithm;
and wherein:
- the second analysis algorithm is an updated version of the first analysis algorithm, and
- the first analysis algorithm and the second analysis algorithm are a band filter, a reconstruction from raw data, and/or a quantitative evaluation.

A method for analysing image data of a patient by means of a server having a database for analysis algorithms is provided, comprising the steps:
- providing a first image data set of a patient,
- performing an analysis of the first image data set by using a first analysis algorithm,
- adding a second analysis algorithm to the database,
- providing a second image data set,
- assigning the second image data set to the patient
- selecting the first analysis algorithm from the database for performing the analysis of the second image data set.

Contrary to the state of the art, it is advantageously possible according to the present invention to use the first analysis algorithm for both the first image data set and the second image data set, even if the second analysis algorithm is available at the time of providing the second image data set. Preferably, the first analysis algorithm and the second analysis algorithm are intended for postprocessing the first and second image data set, for example for measuring a volume of a lesion. The first analysis algorithm and the second analysis algorithm are a band filter, a reconstruction from row data, and/or a quantitative evaluation. Providing the first and the second image data set includes recording an image by means of an imaging instrument being a CT-, NMR-, PET- or X-Ray-instrument. It is conceivable that the server is separated in space from a workstation executing the first and the second analysis algorithm. Preferably, the server is configured as cloud and/or as part of a network. Furthermore, it is provided, that there is a processor device included in the workstation for executing the first and/or second analysis algorithm. It is provided, that adding the second analysis algorithm is performed after analysing the first image data set by using the first analysis algorithm. Furthermore, it is provided, that the first analysis algorithm concerns the same output as the second analysis algorithm. For example, both the first and the second analysis algorithm output the volume of a lesion. Preferably, the second analysis algorithm is an improved or upgraded version of the first analysis algorithm. According to the invention, the second analysis algorithm is an updated version of the first analysis algorithm. In particular, the second image data set is analysed by the first analysis algorithm, although the second analysis algorithm is an improved version of the first analysis algorithm. For example, the second analysis algorithm comprises an improved method for correcting a partial volume compared to the first analysis algorithm. By adding the second analysis algorithm to the database it is further advantageously possible to provide the second analysis algorithm and make use of its benefits at the same time. That means, the user does not have to waive of the advantages of the second analysis algorithm.

According to the invention, it is provided, that
- a first container comprising the first analysis algorithm and a first runtime environment and
- a second container comprising the second analysis algorithm and a second runtime environment are saved in the database. For example the first and/or second container respectively wrap up a piece of software in a complete filesystem that contains everything the first analysis algorithm or second analysis algorithm needs to run: code, runtime, system tools, system libraries - anything that can be installed on the workstation. This guarantees that the first analysis algorithm and/or the second analysis algorithm will always run the same way, regardless of the environment it is running in. Thereby it is possible to run the first analysis algorithm and/or the second analysis algorithm at the workstation independently form the operation system used by the workstation. Consequently, the first analysis algorithm can even be executed by the workstation, even in the case the operation system of the workstation has changed in the meantime. Thereby, the use of containers or a container structure simplifies transferring and installing of the first analysis algorithm. Another advantage of using a container, in particular a closed container, is that there is no need for starting another operation system at the workstation.

According to an alternative, which is not comprised in the scope of the invention, it is provided that
- a first virtual machine is saved along with first analysis algorithm and/or
- a second virtual machine is saved along with the second analysis algorithm to the database. Preferably, a virtual machine (VM) is an emulation of an operation system. Virtual machines are based on computer architectures and provide functionality of a physical computer. By providing the virtual machine it is possible to execute the first analysis algorithm on the workstation, even if the operation system on the workstation has changed.

Expediently, it is provided that the first image data set and/or the second image data set is marked for assigning the first image data set and/or second image data set to the patient. Thus, it is possible to assign the respective first analysis algorithm to the patient and select the proper first analysis algorithm for analysing the second image data set. The first image data set is saved to a memory device, in particular to a picture archiving and communication system (PACS), along with a piece of information about the first analysis. The piece of information might be the version of the analysis algorithm. Further, the memory device might be separated from the workstation in space. Preferably, the piece of information about the first analysis algorithm is incorporated into the digital imaging and communications in medicine (DICOM) that is used as a standard for handling, storing, printing, and transmitting pieces of information in medical imaging. It is also possible to incorporate the piece of information about the first analysis algorithm into a RIS (Radiology information system), a KIS (hospital information system) or other IT-systems. By marking the first image data set and/or second image data set it is possible to identify the first analysis algorithm that was used in the past for performing the analysis of the first image data set.

Preferably, it is provided according to another embodiment of the present invention that the first container is automatically selected from the database for performing the analysis of the second image data set. According to an alternative, which is not comprised in the scope of the invention, the first virtual machine is automatically selected from the database for performing the analysis of the second image data set. Thus, there is no further activity of the user needed for selecting the first analysis algorithm. The workstation identifies the first analysing algorithm corresponding to the patient and selects the first container for analysing the second image data set. It is also conceivable, that the output of the analysis by the first analysis algorithm is compared to the output of the analysis by the second analysis algorithm and a potential difference between the outputs is provided, in particular displayed on a screen of the workstation, automatically. Thus, the user is immediately informed about a change of the outputs or the observed malformation.

According to the present invention it is provided that the first container is transferred from the database to a workstation. The first virtual machine can be transferred from the database to a workstation. Thus, the first analysis algorithm can be executed on the workstation. The workstation has a processor being configured for accessing to the server, in particular to the database, and for executing the first analysis algorithm. It is also conceivable that only the first analysis algorithm is electively transferred to the workstation in the case that the operation system or the version of the operation system has not changed since performing the analysis of the first image data set, even though this is not within the scope of invention. Thus, a relatively small amount of data needs to be transferred. It is also conceivable that the workstation performing the analysis of the first image data set and the workstation performing the analysis of the second image data set differ.

In another embodiment it is provided that the server is shared by several workstations. Thus, several workstations can use the same server. That means it is not necessary to realize and update a database for each workstation. Furthermore, several workstations share one common memory device for saving the first image data set along with the piece of information about the first analysis algorithm. In particular, "sharing" means in this context that several workstations have access to one common server and/or one common memory device. Preferably, the one common server is separated from the common memory device in space. It is also conceivable that several hospitals share on common server, whereas each hospital only has access to its memory device for saving the first image data set along with the piece of information about the first analysis algorithm.

Preferably, the first analysis algorithm and the second analysis algorithm are selected from the database for performing an analysis of the second image data set. Thus, the user can use both the first analysis algorithm for getting a comparable result to the analysis of the first image data set and the second analysis algorithm for getting a more detailed or improved analysis. For example, the user can choose between applying the first analysis algorithm and the second analysis algorithm. It is also conceivable that the method outputs both the result from the first analysis algorithm and the second analysis algorithm.

Another aspect of the present invention is a system for performing a method according to the present invention comprising a server having a database for analysis algorithms. The system is configured to perform a method according to one of the claims 1 to 4.

All of the features described for the method according to the invention and its advantages can be analogously also transferred to the system of the invention and vice versa. According to the present invention it is provided that the system has a workstation and an imaging instrument being a CT-, NMR-, PET- or X-ray-instrument.

Another aspect of the present invention is a computer readable medium having stored thereon computer executable instructions causing a system according to claim 5 to perform a method according to one of the claims 1 to 4. All of the features described for the method according to the invention and its advantages can be analogously also transferred to the computer readable medium of the invention and vice versa.

Further advantages and features will emerge from the following description of preferred embodiments of the method for analysing according to the invention with reference to the accompanying drawings.

In the drawings:

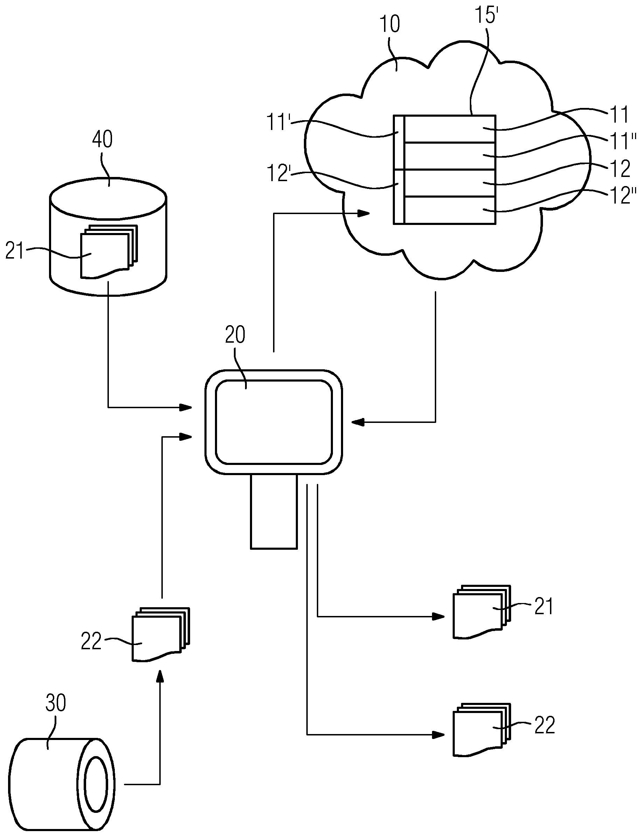

FIG. 1 shows schematically a preferred embodiment of a method for analysing image data of a patient according to a preferred embodiment.

In Figure 1 a method for analysing image data of a patient according to a preferred embodiment is schematically illustrated. Such methods are intended for automatically interpreting and/or categorizing the content of an image data set, such as an X-ray image, a PET-scan image, and/or an NMR-scan image. For example, the method for analysing image data measures the size and/or localizes a malformation being visible in the image data set of the patient. In a first step of the illustrated method a first image data set 21 of the patient is provided by an imaging instrument 30, such as a CT-, NMR-, PET- or X-ray-instrument. Subsequently, the first image data set 21 is analysed by using a first analysis algorithm 11 at a workstation 20 having a processor for running the first analysis algorithm 11 in a second step. Thus, the attending doctor or user gets an output based on the first analysis algorithm. For example, the first analysis algorithm 11 automatically shows that a pulmonary node has a volume of 3,4 cm³. Afterwards the first image data set 21 is saved in a memory device 40, preferably in a picture archiving and communication System (PACS), in a third step. In particular, a piece of information about the first analysis algorithm 11 is saved along with the first image data set 21. Preferably, a piece of information about the first analysis algorithm 11 is incorporated into the Digital Imaging and Communications in Medicine (DICOM) that is used as a standard for handling, storing, printing, and transmitting pieces of information in medical imaging. It is also possible to incorporate the piece of information about the first analysis algorithm 11 into a RIS (Radiology information system), a KIS (hospital information system) or other IT-systems. Due to the development of the operation systems used by the workstation 20 or due to upgrades of the analysis algorithm it cannot guaranteed that analysing a second image data set 22 by using a second analysis algorithm 12 leads to the same result as analysing a second image data set 22 by using the first analysis algorithm 11, wherein a time lag is between recording and/or analysing the first image data set 21 on one hand and recording and/or analysing a second image data set 22 on the other hand. Consequently, the output of the first analysis algorithm 11 cannot compared to the output of the second analysis algorithm 12. For guaranteeing that the outputs of the first analysis algorithm 11 and the second analysis algorithm 12 are comparable a server 10 having a database 15 for analysis algorithms is provided. For example, the server 10 is configured as cloud. In particular, the first analysis algorithm 11 and the second analysis algorithm 12 are stored on the database. Preferably, the first analysis algorithm 11 is available on the server 10 during the first and the second step, while the second analysis algorithm 12 is not available during the first and the second step and is added in a fourth step to the database 15 after the second step and/or the third step. Thus, it is possible to use the first analysis algorithm 11 for analysing the second image data set 22. Preferably, the first analysis algorithm 11 is stored in a closed first container 11' comprising a first run time environment 11'' and/or the second analysis algorithm 12 is stored in a second container 12' comprising a second run time environment 12'' . As a consequence, the first analysis algorithm 11 can advantageously run on a workstation 20 that might no longer compatible with the first analysis algorithm 11. For performing the analysis of the second image data set 22 it is provided that the second image date 22 set is assigned to the patient in a fifth step. Based on the piece of information saved along with the first image data set 21 it is advantageously possible to select the first analysis algorithm 11 or the first container 11' from the database 15 for analysing the second image data set 22. Preferably, the first analysis algorithm 11 or the first container 11' is transferred from the server 10 to the workstation 20 or the workstation 20 runs the first analysis algorithm 11 by accessing the server in a sixth step. Thus, both the first image data set 21 and the second image data set 22 can be analysed by the same first analysis algorithm 11. For example, the first analysis algorithm 11 applied to the second image data set 22 shows that the size of the pulmonary nodules recognized in the first image data 21 set remains the same and the attending doctor comes to the conclusion that there is no cancer.

## Claims

1. Method for analysing image data of a patient intended for automatically interpreting and/or categorizing the content of an image data set:
- in a first step a first image data set (21) of the patient is provided by an imaging instrument (30) being a CT-, NMR-, PET- or X-ray-instrument;
- subsequently, the first image data set (21) is analysed by using a first analysis algorithm (11) at a workstation (20) having a processor for running the first analysis algorithm (11) in a second step;
- afterwards, in a third step, the first image data set (21) is saved in a memory device (40) and a piece of information about the first analysis algorithm (11) is saved along with the first image data set (21);
- a server (10) having a database (15) for analysis algorithms is provided,
- wherein the first analysis algorithm (11) and a second analysis algorithm (12) are stored on the database (15) and the first analysis algorithm (11) is available on the server (10) during the first and the second step, while the second analysis algorithm (12) is not available during the first and the second step,
- and wherein the first analysis algorithm (11) is stored in a closed first container (11') comprising a first run time environment (11") and the second analysis algorithm (12) is stored in a second container (12') comprising a second run time environment (12"),
- the second analysis algorithm (12) is added in a fourth step to the database (15) after the second step and/or the third step,
- for performing the analysis of a second image data set (22), the second image data set (22) is assigned to the patient in a fifth step;
- based on the piece of information saved along with the first image data set (21), selecting the first container (11') from the database (15) for analysing the second image data set (22), wherein the first container (11') is transferred from the server (10) to the workstation (20) in a sixth step, thereby analysing the second image data set (22) with the first analysis algorithm (11);
and wherein:
- the second analysis algorithm (12) is an updated version of the first analysis algorithm (11), and
- the first analysis algorithm (11) and the second analysis algorithm (12) are a band filter, a reconstruction from raw data, and/or a quantitative evaluation.

2. Method according to claim 1, wherein
the first image data set (21) and/or the second image data (22) set is marked for assigning the first image data set (21) and/or second image data set (22) to the patient.

3. Method according to one of the preceding claims, wherein the first container (11') is automatically selected from the database (15) for performing an analysis of the second image data set (22).

4. Method according to one of the preceding claims, wherein the server (10) is shared by several workstations (15).

5. System comprising a server (10) having a database (15) for analysis algorithms, a workstation (20) and an imaging instrument (30) being a CT-, NMR-, PET- or X-ray-instrument, wherein the system is configured to perform a method according to one of the preceding claims.

6. A computer readable medium having stored thereon computer executable instructions causing a system according to claim 5 to perform a method according to one of the claims 1 to 4.

## Patentansprüche

1. Verfahren zur Analyse von Bilddaten eines Patienten, vorgesehen für die automatische Interpretation und/oder Kategorisierung von Inhalten eines Bilddatensatzes:
- in einem ersten Schritt wird ein erster Bilddatensatz (21) des Patienten von einem Bildgebungsinstrument (30) bereitgestellt, bei dem es sich um ein CT-, ein NMR-, ein PET- oder ein Röntgeninstrument handelt;
- anschließend wird der erste Bilddatensatz (21) analysiert, indem ein erster Analysealgorithmus (11) an einer Arbeitsstation (20) verwendet wird, die über einen Prozessor zum Ausführen des ersten Analysealgorithmus (11) in einem zweiten Schritt verfügt;
- danach wird, in einem dritten Schritt, der erste Bilddatensatz (21) in einer Speichervorrichtung (40) gespeichert, und eine Information zu dem ersten Analysealgorithmus (11) wird zusammen mit dem ersten Bilddatensatz (21) gespeichert;
- ein Server (10), der über eine Datenbank (15) für Analysealgorithmen verfügt, wird bereitgestellt,
- wobei der erste Analysealgorithmus (11) und ein zweiter Analysealgorithmus (12) in der Datenbank (15) gespeichert sind und der erste Analysealgorithmus (11) während des ersten und des zweiten Schritts auf dem Server (10) verfügbar ist, während der zweite Analysealgorithmus (12) während des ersten und des zweiten Schritts nicht verfügbar ist,
- und wobei der erste Analysealgorithmus (11) in einem geschlossenen ersten Container (11') gespeichert ist, der eine erste Laufzeitumgebung (11") umfasst, und der zweite Analysealgorithmus (12) in einem zweiten Container (12') gespeichert ist, der eine zweite Laufzeitumgebung (12") umfasst,
- der zweite Analysealgorithmus (12) wird in einem vierten Schritt in die Datenbank (15) aufgenommen, nach dem zweiten Schritt und/oder dem dritten Schritt,
- zum Durchführen der Analyse eines zweiten Bilddatensatzes (22) wird der zweite Bilddatensatz (22) dem Patienten in einem fünften Schritt zugewiesen;
- basierend auf der Information, die zusammen mit dem ersten Bilddatensatz (21) gespeichert wird, Auswählen des ersten Containers (11') aus der Datenbank (15) zum Analysieren des zweiten Bilddatensatzes (22), wobei der erste Container (11') in einem sechsten Schritt von dem Server (10) an die Arbeitsstation (20) übertragen wird, wodurch der zweite Bilddatensatz wird (22) mit dem ersten Analysealgorithmus (11) analysiert;
und wobei:
- der zweite Analysealgorithmus (12) eine aktualisierte Version des ersten Analysealgorithmus (11) ist, und
- der erste Analysealgorithmus (11) und der zweite Analysealgorithmus (12) ein Bandfilter, eine Rekonstruktion anhand von Rohdaten und/oder eine quantitative Auswertung sind.

2. Verfahren gemäß Anspruch 1, wobei
der erste Bilddatensatz (21) und/oder der zweite Bilddatensatz (22) für ein Zuweisen des ersten Bilddatensatzes (21) und/oder des zweiten Bilddatensatzes (22) zu dem Patienten markiert sind.

3. Verfahren gemäß einem der vorstehenden Ansprüche, wobei der erste Container (11') automatisch aus der Datenbank (15) ausgewählt wird, um eine Analyse des zweiten Bilddatensatzes (22) durchzuführen.

4. Verfahren gemäß einem der vorstehenden Ansprüche, wobei der Server (10) von mehreren Arbeitsstationen (15) gemeinsam genutzt wird.

5. System, umfassend einen Server (10), der über eine Datenbank (15) für Analysealgorithmen verfügt, eine Arbeitsstation (20) und ein Bildgebungsinstrument (30), bei dem es sich um ein CT-, ein NMR-, ein PET- oder ein Röntgeninstrument handelt, wobei das System dazu ausgelegt ist, ein Verfahren gemäß einem der vorstehenden Ansprüche durchzuführen.

6. Computerlesbares Medium, auf dem computerausführbare Anweisungen gespeichert sind, die ein System gemäß Anspruch 5 veranlassen, ein Verfahren gemäß einem der Ansprüche 1 bis 4 durchzuführen.

## Revendications

1. Procédé d'analyse de données d'image d'un patient en vue d'interpréter et/ou de catégoriser automatiquement le contenu d'un ensemble de données d'image :
- dans un premier stade, un premier ensemble (21) de données d'image du patient est fourni par un instrument (30) d'imagerie, qui est un instrument CT, RMN, PET ou à rayons X ;
- ensuite, le premier ensemble (21) de données d'image est analyser en utilisant un premier algorithme (11) d'analyse à un poste (20) de travail ayant un processeur pour exécuter le premier algorithme (11) d'analyse dans un deuxième stade ;
- après quoi, dans un troisième stade, le premier ensemble (21) de données d'image est sauvegardé dans un dispositif (40) de mémoire et une information sur le premier algorithme (11) d'analyse est sauvegardé en même temps que le premier ensemble (21) de données d'image ;
- il est prévu un serveur (10) ayant une base (15) de données pour des algorithmes d'analyse,
- dans lequel le premier algorithme (11) d'analyse et un deuxième algorithme (12) d'analyse sont mis en mémoire sur la base (15) de données et le premier algorithme (11) d'analyse est disponible sur le serveur (10) pendant le premier et le deuxième stade, tandis que le deuxième algorithme (12) d'analyse n'est pas disponible pendant le premier et le deuxième stade,
- et dans lequel le premier algorithme (11) d'analyse est mis en mémoire dans un premier récipient (11') fermé comprenant un premier environnement (11") de temps de fonctionnement, et le deuxième algorithme (12) d'analyse est mis en mémoire dans un deuxième récipient (12') comprenant un deuxième environnement (12") de temps de fonctionnement,
- le deuxième algorithme (12) d'analyse est ajouté dans un quatrième stade à la base (15) de données, après le deuxième stade et/ou le troisième stade,
- pour effectuer l'analyse d'un deuxième ensemble (22) de données d'image, le deuxième ensemble (22) de données d'image est affecté au patient dans un cinquième stade ;
- sur la base de l'information sauvegardée en même temps que le premier ensemble (21) de données d'image, on sélectionne le premier récipient (11') dans la base (15) de données pour analyser le deuxième ensemble (22) de données d'image, le premier récipient (11') étant transféré du serveur (10) au poste (20) de travail dans un sixième stade, en analysant ainsi le deuxième ensemble (22) de données d'image par le premier algorithme (11) d'analyse ; et dans lequel
- le deuxième algorithme (12) d'analyse est une version mise à jour du premier algorithme (11) d'analyse, et
- le premier algorithme (11) et le deuxième algorithme(12) d'analyse sont un filtrage de bande, une reconstruction à partir de données brutes et/ou une évaluation quantitative.

2. Procédé suivant la revendication 1, dans lequel
le premier ensemble (21) de données d'image et/ou le deuxième ensemble (22) de données d'image est marqué pour affecter le premier ensemble (21) de données d'image et/ou le deuxième ensemble (22) de données d'image au patient.

3. Procédé suivant l'une des revendications précédentes, dans lequel
le premier récipient (11') est sélectionné automatiquement dans la base (15) de données pour effectuer une analyse du deuxième ensemble (22) de données d'image.

4. Procédé suivant l'une des revendications précédentes, dans lequel
le serveur (10) est partagé par plusieurs postes (15) de travail.

5. Système comprenant un serveur (10) ayant une base (15) de données pour des algorithmes d'analyse, un poste (20) de travail et un instrument (30) d'imagerie, qui est un instrument CT, RMN, PET ou à rayons X, dans lequel le système est configuré pour effectuer un procédé suivant l'une des revendications précédentes.

6. Support déchiffrable par ordinateur, sur lequel sont mises en mémoire des instructions exécutables par ordinateur, faisant qu'un système suivant la revendication 5 effectue un procédé suivant l'une des revendications 1 à 4.
